# FASCICULE DE BREVET EUROPEEN

(11) **EP 3 744 401 B1**
(45) Date de publication et mention de la délivrance du brevet: **14.06.2023**
(21) Numéro de dépôt: 19305673.6
(22) Date de dépôt: 28.05.2019
(51) Int. Cl.: A61Q 19/00, A61K 36/8967, A61Q 19/08, C12N 1/18, C12R 1/865, A61K 8/9794

(54) **PROCÉDÉ D' EXTRACTION DE VÉGETAUX COMPRENANT UNE ÉTAPE DE FERMENTATION AVEC SACCHAROMYCES CEREVISIAE**
PFLANZENEXTRAKTIONSVERFAHREN ENTHALTEND EINEN FERMENTATIONSSCHRITT MIT SACCHAROMYCES CEREVISIAE
METHOD FOR EXTRACTING PLANTS COMPRISING A STEP OF FERMENTATION WITH SACCHAROMYCES CEREVISIAE

(43) Date de publication de la demande: 02.12.2020
(73) Titulaire: Chanel Parfums Beauté, 92200 Neuilly-sur-Seine (FR)
(72) Inventeur: TORIBIO, ALIX, 93694 PANTIN CEDEX (FR); LEGANGNEUX, DAVID, 93694 PANTIN CEDEX (FR); COCANDEAU, VINCENT, 93694 PANTIN CEDEX (FR)
(74) Mandataire: Plasseraud IP

(56) Documents cités:
- CN-A- 101 243 897
- FR-A1- 2 929 852
- FR-A1- 3 026 946
- KR-A- 20100 006 796
- KR-B1- 100 815 256
- LEE PAO-JU ET AL: "Analysis of NO-suppressing activity of Strawberry Wine supplemented with ball-milled achenes", JOURNAL OF FOOD SCIENCE AND TECHNOLOGY, SPRINGER (INDIA) PRIVATE LTD, INDIA, vol. 55, no. 4, 3 février 2018 (2018-02-03), pages 1285-1294, XP036469903, ISSN: 0022-1155, DOI: 10.1007/S13197-018-3039-8 [extrait le 2018-02-03]
- BRGLEZ MOJZER EVA ET AL: "Polyphenols: Extraction Methods, Antioxidative Action, Bioavailability and Anticarcinogenic Effects.", MOLECULES (BASEL, SWITZERLAND) 11 JUL 2016, vol. 21, no. 7, 11 juillet 2016 (2016-07-11), XP002795595, ISSN: 1420-3049
- Unknown: "Quercetin", BulkActives Ingredients for skin care , 20 mars 2015 (2015-03-20), XP002795596, Extrait de l'Internet: URL:https://www.bulkactives.com/product/pr oduct/quercetin.html [extrait le 2019-11-12]
- Unknown: "IOC 18-2007(TM)", Lallemand Wine catalogue, 21 mai 2018 (2018-05-21), XP055642029, Extrait de l'Internet: URL:https://catalogapp.lallemandwine.com/u ploads/yeasts/docs/253a40560c756452127fa09 a6a24a3b9e0b4592a.pdf [extrait le 2019-11-13]
- Anonymous: "IOC Divine(TM) | Catalogue | Lallemand Wine", Lallemand wine catalogue, 2 mai 2013 (2013-05-02), XP055642035, Extrait de l'Internet: URL:https://www.lallemandwine.com/en/austr alia/products/catalogue/wine-yeasts/100/io c-divine/ [extrait le 2019-11-13]
- Anonymous: "IOC Fizz+ (TM)", Lallemand wine catalogue, 2 mai 2013 (2013-05-02), XP055642039, Extrait de l'Internet: URL:https://catalogapp.lallemandwine.com/u ploads/yeasts/docs/e2926b6a7b4c9708ccc5b9d b0d2517b5e9e760a2.pdf [extrait le 2019-11-13]

## Description

### Domaine de l'invention

La présente invention a pour objet un procédé pour la préparation d'un extrait végétal enrichi en polyphénols et/ou substantiellement dépourvu de sucres simples.

### Arrière-plan technique de l'invention

La peau est principalement constituée de trois couches, à savoir, en partant de la plus superficielle, l'épiderme, le derme et l'hypoderme.

L'épiderme est en particulier constitué de kératinocytes (majoritaires), de mélanocytes (intervenant dans la pigmentation de la peau) et de cellules de Langerhans. Sa fonction est de protéger le corps de l'environnement extérieur et d'assurer son intégrité, et notamment de freiner la pénétration de micro-organismes ou de substances chimiques, et d'empêcher l'évaporation de l'eau contenue dans la peau.

Pour ce faire, les kératinocytes subissent un processus de maturation orientée continu au cours duquel les kératinocytes situés dans la couche basale de l'épiderme forment, au stade terminal de leur différenciation, des cornéocytes qui sont des cellules mortes totalement kératinisées sous forme d'enveloppes cornées constituées de protéines et de lipides tels que des céramides. Lors de ce processus de différenciation, des lipides épidermiques intercornéocytaires sont en outre formés puis organisés sous forme de bicouches (feuillets) dans le *stratum corneum.* Ils participent, avec les enveloppes cornées précitées, à la fonction barrière de l'épiderme.

La fonction barrière de l'épiderme peut toutefois se trouver perturbée dans certaines conditions climatiques (sous l'effet du froid et/ou du vent, par exemple), ou encore sous l'effet du stress ou de la fatigue, notamment, favorisant ainsi la pénétration d'allergènes, d'agents irritants ou de micro-organismes qui occasionnent ainsi un dessèchement cutané susceptible de générer des sensations d'inconfort telles que des tiraillements ou des rougeurs, et également d'altérer l'éclat du teint et la souplesse de la peau.

Pour prévenir ce phénomène ou le corriger, il est connu d'appliquer sur la peau des compositions cosmétiques renfermant des agents hygroscopiques, tels que des sucres ou des polyols, destinés à capter l'eau présente dans la peau et à freiner ainsi son évaporation. Généralement, ces compositions incluent en outre des actifs agissant sur une ou plusieurs cibles biologiques intervenant par exemple dans les processus de vieillissement cutané.

En raison d'une volonté toujours plus grande des consommateurs de se tourner vers des produits naturels renfermant le moins d'ingrédients synthétiques possibles, et au vu des contraintes réglementaires de plus en plus lourdes pesant sur les composés issus de l'industrie chimique, les actifs issus d'extraits végétaux sont aujourd'hui privilégiés.

Les extraits végétaux ont pour avantage de contenir de nombreux polyols et particulièrement des polyphénols, dont les effets antioxydants sont maintenant largement reconnus.

Cependant, les extraits végétaux contiennent également de nombreux sucres simples (représentant 25 à 75% en poids des extraits conventionnels) tels que le glucose, le fructose et le galactose, qui ont pour effet de nuire à l'activité biologique des actifs végétaux.

Afin de réduire leur teneur en sucres, les extraits végétaux sont généralement traités à l'aide de techniques de purification telles que l'utilisation de résines, de charbon actif, de cellulose ou par distillation. Ces traitements visent à éliminer la matrice polaire contenant les sucres simples.

Toutefois, ces techniques sont aspécifiques et éliminent également d'autres molécules intéressantes telles que les acides aminés ou les acides organiques.

Aussi, il existe un vrai besoin pour procédé d'extraction permettant d'obtenir des extraits végétaux qui contiennent de nombreux polyphénols dont les pouvoirs hydratants et antioxydants sont aujourd'hui largement reconnus, mais qui soient également dépourvus de sucres simples tels que le glucose, le fructose et le galactose qui nuisent à l'activité biologique des extraits.

### Résumé de l'invention

La demanderesse est parvenue à mettre au point un procédé pour la préparation d'un extrait végétal enrichi en polyphénols et substantiellement dépourvu de sucres simples. La demanderesse a en effet démontré qu'il était possible d'éliminer les sucres simples et d'augmenter la teneur en polyphénols d'un extrait végétal en effectuant une étape de fermentation dudit extrait par une levure du type *Saccharomyces cerevisiae* var. *bayanus.*

La demanderesse a en outre démontré que l'utilisation spécifique de cette variété de levures permettait d'obtenir une cinétique de fermentation optimale, avec une consommation rapide et totale des sucres simples contenus dans les extraits végétaux.

La présente invention porte donc sur un procédé pour la préparation d'un extrait végétal enrichi en polyphénols et/ou substantiellement dépourvu de sucres simples, comprenant une étape de fermentation d'un extrait végétal par une levure appartenant à la variété *Saccharomyces cerevisiae* var. *bayanus.*

Un extrait végétal susceptible d'être obtenu selon le procédé décrit ci-dessus a pour particularité d'être substantiellement dépourvu de sucres simples mais aussi de contenir de fortes teneurs en polyphénols tels que les flavonoïdes.

### Description détaillée de l'invention

La présente invention porte sur un procédé pour la préparation d'un extrait végétal enrichi en polyphénols et/ou substantiellement dépourvu de sucres simples, ledit procédé comprenant une étape de fermentation d'un extrait végétal par une levure appartenant à la variété *Saccharomyces cerevisiae* var. *bayanus.*

Les polyphénols sont des molécules comprenant au moins deux groupes phénoliques, c'est-à-dire comprenant au moins deux noyaux benzéniques portant des fonctions hydroxyle. Parmi les polyphénols, on peut notamment citer les acides hydroxybenzoïques, les acides hydroxycinnamiques et coumarines, les naphtoquinones, les stilbènoïdes ou encore les flavonoïdes. Les flavonoïdes sont les polyphénols les plus représentés dans le règne végétal, ils comprennent particulièrement les flavones, flavonols et dihydroflavonols, flavanones, aurones, chalcones et dihydrochalcones, flavanols, flavanediols, anthocyanidines, et leurs hétérosides. Les polyphénols sont reconnus pour leurs effets antioxydants, qui en font des actifs anti-âge particulièrement intéressants. De plus, la présence de nombreux groupes hydroxyles dans les polyphénols permet de capter l'eau présente dans la peau et de freiner ainsi son évaporation. Les polyphénols ont donc également des effets hydratants très intéressants.

Par un extrait « enrichi en polyphénols » on entend ici un extrait comprenant de 5.00 à 100% de polyphénols en poids, particulièrement de 5.00 à 85.00% de polyphénols en poids, plus particulièrement de 55.00 à 85.00% de polyphénols en poids.

Selon la présente invention, les « sucres simples » sont le glucose, le fructose, le galactose et le mannose, mais également les dimères de glucose, de fructose, de galactose et de mannose.

Dans le cadre de la présente invention, un extrait « substantiellement dépourvu de sucres simples » est un extrait comprenant moins de 20.00% en poids, particulièrement moins de 10.00% en poids, préférentiellement moins de 5.00% en poids, de manière encore plus préférée moins de 1.00% en poids de sucres simples.

Dans un mode de réalisation particulier, l'extrait obtenu est complètement dépourvu de sucres simples, c'est-à-dire qu'il comprend 0% en poids de sucres simples.

Le présent procédé comprend une étape de fermentation d'un extrait végétal par une levure appartenant à la variété *Saccharomyces cerevisiae* var. *bayanus.* Cette étape de fermentation permet de réduire voire d'éliminer les sucres simples contenus dans un extrait végétal, mais aussi d'enrichir la teneur de l'extrait végétal en polyphénols.

La demanderesse a démontré (voir exemples ci-dessous) que l'utilisation d'une levure de la variété *Saccharomyces cerevisiae* var. *bayanus* permettait d'obtenir une cinétique de fermentation optimale pour les extraits végétaux, et surtout bien meilleure que celle obtenue en utilisant d'autres variétés de levures. Les levures *Saccharomyces cerevisiae* var. *bayanus* permettent particulièrement de diminuer le temps de fermentation des extraits végétaux, c'est-à-dire le temps nécessaire à la consommation des sucres dans l'extrait, à moins de 24H. Ces levures permettent donc de consommer rapidement et efficacement les sucres simples contenus dans les extraits végétaux.

Les levures de la variété *Saccharomyces cerevisiae* var. *bayanus* sont connues et particulièrement utilisées dans le domaine de l'œnologie. L'homme du métier sait comment obtenir de telles levures pour mettre en œuvre la présente invention. Dans un mode de réalisation particulier, la levure *Saccharomyces cerevisiae* var. *bayanus* est choisie parmi les souches de levures commercialisées sous les références IOC Fizz+, IOC Divine, IOC 18-2007 ou un mélange de celles-ci. Toutes ces souches sont référencées par l'institut français de la vigne et du vin et facilement accessibles pour l'homme du métier.

La souche de levures IOC Fizz+ est un mélange de deux levures déposées à l'Institut Pasteur sous les références LYCC 6022 : LA CLAIRE CGC62 et LYCC 6039 : LA CLAIRE SP665.

La souche IOC Divine correspond quant à elle à la souche déposée à l'Institut Pasteur sous la référence LYCC 7000, et la souche 18-2007 à celle déposée à l'Institut Pasteur sous la référence CNCM I-5320.

Les souches de levures selon la présente invention peuvent être sous forme sèche, sous forme liquide ou sous forme de crème de levures.

Dans un mode de réalisation particulier, la souche de *Saccharomyces cerevisiae* var. *bayanus* est sous forme sèche.

Typiquement, l'étape de fermentation se fait en utilisant la souche de *Saccharomyces cerevisiae* var. *bayanus* à entre 1 et 5% en masse par rapport à la solution à fermenter, particulièrement à entre 2 et 3% en masse par rapport à la solution à fermenter, et plus particulièrement à 2% en masse par rapport à la solution à fermenter.

La durée de l'étape de fermentation doit être suffisante pour permettre d'atteindre un taux de sucres simples inférieur à 20% en poids, particulièrement 10% en poids, préférentiellement inférieur à 5% en poids, de manière encore plus préférée inférieur à 1% en poids dans l'extrait végétal. Typiquement, l'étape de fermentation est conduite sur une période de 1 à 72 heures, particulièrement de 12 à 60 heures, de manière préférée de 18 à 48 heures.

Dans un mode de réalisation particulier, l'étape de fermentation peut être effectuée au moyen d'un levain. Ce levain est préparé à partir de la fermentation d'un extrait végétal concentré (ledit extrait végétal étant le même que celui utilisé dans le procédé de l'invention) avec la souche de *Saccharomyces cerevisiae* var. *bayanus.* L'extrait végétal utilisé pour le levain est typiquement concentré jusqu'à obtenir un taux de matière sèche de l'ordre de 5 à 30%.

Selon ce mode de réalisation, l'étape de fermentation du procédé selon l'invention est effectuée en ajoutant ce levain à l'extrait végétal. L'utilisation d'un levain permet d'amorcer la fermentation et d'améliorer ainsi la cinétique de l'étape de fermentation du procédé selon l'invention.

Typiquement, le levain est préparé en fermentant pendant environ 2 heures à température ambiante (ou alternativement à 37°C) un extrait végétal concentré à entre 5 et 50%, particulièrement à entre 15 et 30% de matière sèche dans l'eau avec 10% en poids de levures sèches *Saccharomyces cerevisiae var. bayanus.* 1 à 5% de ce levain en poids, préférentiellement 2% de levain en poids, sont ajoutés à l'extrait végétal pour l'étape de fermentation selon le procédé de l'invention.

L'extrait végétal utilisé selon le procédé de l'invention peut être obtenu selon n'importe quel procédé d'extraction connu de l'homme du métier.

Typiquement, un procédé de préparation d'un extrait végétal comprend les étapes suivantes:
a) l'extraction des plantes avec au moins un solvant alcoolique et/ou de l'eau ;
b) la filtration, par exemple par tamisage, du mélange obtenu en a) afin d'éliminer les résidus végétaux,
c) optionnellement, la décoloration du mélange obtenu à l'issue l'étape b; et
d) l'élimination du solvant et la concentration de l'extrait.

Aussi, selon un mode de réalisation, la présente invention porte sur un procédé pour la préparation d'un extrait végétal enrichi en polyphénols et/ou substantiellement dépourvu de sucres simples comprenant les étapes suivantes :
a) l'extraction des plantes avec au moins un solvant alcoolique et/ou de l'eau ;
b) la filtration du mélange obtenu en a, par exemple par tamisage, afin d'éliminer les résidus végétaux ;
c) optionnellement, la décoloration du mélange obtenu à l'issue l'étape b;
d) l'élimination du solvant, par exemple par évaporation, et la concentration de l'extrait; et
e) la fermentation de l'extrait obtenu à l'issu de l'étape d) avec une levure appartenant à la variété *Saccharomyces cerevisiae* var. *bayanus.*

La « plante » utilisée à l'étape a) peut être n'importe quel être vivant appartenant au règne végétal. La plante est une plante terrestre ligneuse ou une plante herbacée. De manière préférée, la plante est une phanérogame ou « plante à fleur ». Elle peut, par exemple, être choisie parmi le Lys (*Lilium candidum*), le myosostis (*Myosotis sylvatica*), le sureau (*Sambucus sp.*), le camelia (*Camellia sp.*), la primevère (*Primula sp.*), la tubéreuse (*Polianthes tuberosa*), le jasmin (*Jasminum grandiflorum*), le plantain (*Plantago lanceolata*), le coing (*Cydonia oblonga*), l'abricotier (*Prunus armeniaca*), ou le frangipanier (*Plumeria sp.*).

La partie de la plante extraite à l'étape a) est la fleur. L'extrait végétal selon la présente invention est donc un extrait de fleurs.

La plante utilisée à l'étape a) peut être sous forme « fraîche », c'est-à-dire être utilisé dans les 48 heures, particulièrement les 24 heures, encore plus particulièrement les 12 heures suivant la récolte.

La plante utilisée à l'étape a) peut également être sous forme sèche. Dans ce cas, la plante fraîche est déshydratée en conditions douces, soit à température ambiante à l'abri de la lumière soit dans un séchoir ventilé à une température inférieure à 35°C. La plante est de préférence séchée jusqu'à obtention d'une teneur en matière sèche supérieure à 80% et préférentiellement supérieure à 85%.

L'étape a) peut en outre comprendre le broyage des fleurs afin d'obtenir des particules de taille inférieure à 5 cm, de préférence inférieure à 2 cm.

L'étape d'extraction est effectuée avec au moins un solvant d'extraction comprenant un solvant alcoolique et/ou de l'eau. Le solvant d'extraction dépend de la plante choisie. Le ratio plante/solvant typiquement de 1 pour 10 (poids/poids).

Le solvant d'extraction est de l'eau et/ou un solvant alcoolique. Dans un mode de réalisation particulier, le solvant alcoolique est un alcool tel que l'éthanol, particulièrement l'éthanol à 96°.

Le solvant d'extraction peut contenir de 0 à 100% en volume d'eau et de 0 à 100% en volume de solvant alcoolique. Dans un mode de réalisation particulier, le solvant d'extraction comprend 100% d'eau, dans un autre mode de réalisation, il comprend 100% d'éthanol. Le solvant d'extraction peut également comprendre 50% en volume d'eau et 50% en volume d'éthanol.

Dans un mode de réalisation particulier, l'eau peut être de l'eau déionisée.

L'étape d'extraction dure au moins 1 heure, de préférence au moins 2 heures, particulièrement au moins 3 heures, et peut être renouvelée une à deux fois.

Comme indiqué ci-dessus, le procédé d'extraction peut comprendre une étape dans laquelle le mélange issu de l'étape b) est décoloré. Cette étape vise à éliminer les pigments présents dans l'extrait tels que les chlorophylles et les xanthophylles. L'homme du métier connaît plusieurs méthodes permettant d'éliminer ces pigments. La décoloration peut, par exemple, être effectuée en mettant le mélange en contact avec du charbon actif. Une fois la décoloration effectuée, le mélange est filtré afin d'éliminer les résidus de charbon.

Typiquement, l'étape d) comprend la concentration de l'extrait jusqu'à obtenir un taux de matière sèche dans l'eau de l'ordre 5 à 30%, de manière préférée de 15 à 30%.

L'extrait végétal issu de l'étape d), c'est-à-dire l'extrait végétal concentré, peut être utilisé pour préparer le levain mentionné plus haut.

Le procédé décrit ci-dessus peut en outre comprendre une étape d') entre les étapes d) et e), dans laquelle le mélange contenant l'extrait concentré est finement filtré (typiquement à 2pm) afin d'éliminer les particules fines et les bactéries résiduelles.

Le procédé peut comprendre en outre une étape f), dans laquelle l'extrait fermenté est filtré puis dilué afin d'obtenir un extrait contenant 1 à 10% de matière sèche en poids. Cet extrait peut se présenter sous forme de solution aqueuse limpide et stable.

Dans un mode de réalisation particulier, le procédé selon la présente invention comprend les étapes suivantes :
a) Une poudre de fleurs broyées à une finesse inférieure à 2cm est extraite deux fois avec de l'éthanol, ou de l'eau ou un mélange des deux à 60-75 °C durant au moins 2 heures ;
b) Le mélange est filtré jusqu'à 4pm ;
c) Le mélange résultant est soumis à une décoloration par contact avec du charbon activé pendant une heure puis subit une microfiltration jusqu'à 1µm afin d'éliminer les résidus de charbon ;
d) Elimination du solvant d'extraction par évaporation et concentration de l'extrait jusqu'à atteindre un taux de matière sèche dans l'eau de l'ordre de 5 à 15% (poids/poids) ;
d') Le mélange est filtré à 0.2pm ;
e) Fermentation pendant 24 à 48 heures à 37°C du mélange avec 2% en poids d'un levain, ledit levain ayant été obtenu en concentrant une partie de l'extrait obtenu avant l'étape d) à 30% de matière sèche dans l'eau et en y ajoutant 10% en poids de levures sèches *Saccharomyces cerevisiae var. bayanus* pour une première fermentation durant 2 heures à 37°C ;
f) L'extrait fermenté est ensuite filtré à 1µm, puis dilué pour obtenir un extrait contenant 1 à 10% de matière sèche en poids auquel est ajouté un système de conservation, à base de glycols et/ou autres.

Un extrait végétal susceptible d'être obtenu selon le procédé décrit ci-dessus, c'est-à-dire un extrait végétal enrichi en polyphénols et/ou substantiellement dépourvu de sucres simples est décrit ici mais n'est pas couvert par le texte des revendications.

Un tel extrait végétal est caractérisé en ce qu'il comprend :
- 5.00 à 100.00% de polyphénols en poids, particulièrement de 5.00 à 85.00% de polyphénols en poids, plus particulièrement de 55.00 à 85.00% de polyphénols en poids ; et/ou
- moins de 20.00% en poids, particulièrement moins de 10.00% en poids, préférentiellement moins de 5.00% en poids, de manière encore plus préférée moins de 1.00% en poids de sucres simples.

L'extrait végétal est utilisé à des fins cosmétiques, pour hydrater la peau humaine ou la protéger vis-à-vis du dessèchement. Grâce aux effets antioxydants des polyphénols, l'extrait peut également être utilisé pour lutter contre les signes du vieillissement tels que les rides et ridules, la perte de fermeté et d'élasticité dues à une perte tissulaire au niveau de l'épiderme et/ou du derme ; la perte d'éclat due à la réduction de la microcirculation et à un ralentissement du renouvellement cellulaire au niveau de l'épiderme, l'apparition de taches pigmentaires associées à un dysfonctionnement de la synthèse de la mélanine, ou encore la sécheresse cutanée résultant d'une diminution de la fonction barrière de la couche cornée et à un ralentissement du renouvellement épidermique.

Les antioxydants sont en effet connus pour leur capacité à piéger les radicaux libres qui sont l'un des facteurs majeurs de l'accélération du vieillissement cutané.

Une composition cosmétique comprenant un extrait végétal tel que décrit précédemment est divulguée ici, mais n'est pas couverte par le texte des revendications.

De préférence, ledit extrait est présent dans la composition cosmétique ou dermatologique à raison de 0,001 à 90% en poids total de la composition, en particulier à raison de 0,01 à 10%, de préférence de 0,1 à 10% en poids total de la composition. Ladite composition cosmétique ou dermatologique peut notamment, être adaptée à une application par voie topique.

Avantageusement, ladite composition cosmétique ou dermatologique peut se présenter sous la forme d'une poudre, d'une émulsion, d'une microémulsion, d'une nanoémulsion, d'une suspension, d'une solution d'une lotion, d'une crème, d'un gel aqueux ou hydroalcoolique, d'une mousse, d'un sérum, d'une solution ou d'une dispersion pour aérosol, ou d'une dispersion de vésicules lipidiques.

Dans le cas d'une émulsion, il peut s'agir d'une émulsion eau dans huile ou huile dans eau.

La composition cosmétique ou dermatologique peut comprendre également un solvant choisi en fonction des différents ingrédients et de la forme d'administration.

A titre d'exemples, on peut citer l'eau (de préférence de l'eau déminéralisée), un alcool tel que l'éthanol, ou un éther de diéthylène glycol tel que l'éthoxydiglycol ou l'éther monométhylique de diéthylène glycol.

Ladite composition cosmétique peut également comprendre, en outre de l'extrait, au moins un additif usuel dans le domaine, tel que par exemple au moins un composé choisi parmi un agent émollient ou humectant, un agent gélifiant et/ou épaississant, un agent tensioactif, une huile, un agent actif, un colorant, un conservateur, un agent antioxydant, un agent actif, une poudre organique ou inorganique, un filtre solaire et un parfum.

Notamment, ladite composition peut contenir :
- Un ou plusieurs agent(s) émollient(s) ou humectant(s), qui peuvent être choisi(s) par exemple parmi la glycérine, les glycols, les silicones hydrosolubles tels que celui vendu sous la dénomination KF6011 (Shin Etsu) et le Jojoba hydrosoluble, tel que celui vendu sous la dénomination Resplanta jojoba (Res pharma).
   Ledit agent émollient ou humectant peut être présent dans la composition à une teneur de l'ordre de 0 à 30%, de préférence 2 à 10% en poids, par rapport au poids total de la composition.
- Un ou plusieurs agent(s) gélifiants(s) et/ou épaississant(s) de la phase aqueuse, choisi par exemple parmi les dérivés cellulosiques, gommes d'origine végétale (guar, caroube, alginates, carraghénanes, pectine), d'origine microbienne (xanthane), les argiles (laponite), les matériaux identifiés par les noms INCI "ammonium acryloyldiméthyltaurate/vp copolymer" et "ammonium acryloyldiméthyl-taurate/beheneth-25 méthacrylate copolymer" (tels que par exemple ceux vendus sous les dénominations Aristoflex AVC et HMB par Clariant).
   Ledit agent gélifiant et/ou épaississant peut être présent dans la composition à une teneur de l'ordre de 0 à 10% en poids, par rapport au poids total de la composition.
- Un ou plusieurs agent(s) tensioactif(s), de préférence non ionique, présent dans une teneur de l'ordre de 0 à 8%, de préférence 0,5 à 3% en poids, par rapport au poids total de la composition.
- Un ou plusieurs corps gras liquide(s) à température ambiante, communément dénommé(s) huiles(s), volatil(s) ou non volatile(s), hydrocarboné(s) ou siliconé(s), linéaire(s), cyclique(s) ou ramifié(s), par exemple, l'isododécane, le cyclopentadimethylsiloxane, les diméthicones, l'isononanoate d'isononyle ou le pentaerythrityl tetraisostéarate, de préférence à raison de 0 à environ 10%, de préférence 0,5 à 5% en poids, par rapport au poids total de la composition.

- Un ou plusieurs agent(s) actif(s), d'origine naturelle ou synthétique, ayant une activité biologique, par exemple choisis parmi les vitamines, les oligo-éléments, l'allantoïne, les protéines végétales, les extraits végétaux, les agents hydratants, les agents antiâges, les antioxydants, les agents favorisant l'éclat et des mélanges de ceux-ci. En particulier, l'agent actif est choisi parmi une eau de fruit de vanilla planifolia, le niacinamide, l'acide hyaluronique et ses dérivés, un extrait de levure et des mélanges de ceux-ci.
- Un ou plusieurs colorant(s) hydrosoluble(s) tels que, par exemple, le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine ou la xanthophylle, de préférence à raison de 0 à environ 2% en poids, par rapport au poids total de la composition.

D'autres additifs habituellement utilisés en cosmétique peuvent également être présents dans la composition, notamment des conservateurs, des agents antioxydants ou des parfums bien connus dans le domaine technique.

L'homme du métier est en mesure de choisir, parmi l'ensemble de ces éventuels additifs, aussi bien la nature que la quantité de ceux qui seront ajoutés à la composition, de telle sorte que celle-ci conserve l'ensemble de ses propriétés.

L'invention sera maintenant illustrée par les exemples non limitatifs suivants.

### BREVE DESCRIPTION DES FIGURES

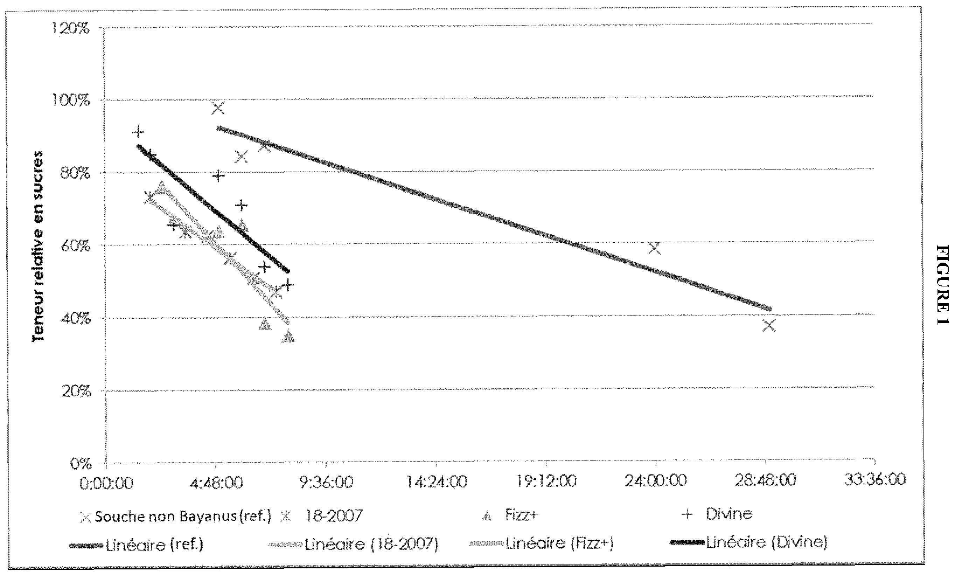

**Figure 1** : Régression linéaire des teneurs en sucres totaux consommées lors de la fermentation pour chacune des souches étudiées *(Souche référence Saccharomyces cerevisiae* n'appartenant pas à la variété *bayanus [ref],* et *les souches Saccharomyces cerevisiae* var. *bayanus 18-2007, Fizz+* et *Divine)*

### EXEMPLES

**EXEMPLE 1 -** Procédé d'extraction de Lys selon la présente invention:
1) Les fleurs fraîches de Lys sont ensuite extraites deux fois avec de l'Ethanol 96° à 60 °C, durant 2 heures minimum, le ratio plante/solvant est de 1 pour 10 (poids/poids) ;
2) Le mélange est tamisé à 100µm afin d'éliminer les résidus végétaux puis laissé au repos une nuit et à nouveau filtré jusqu'à 4pm ;
3) Le mélange résultant est soumis à une décoloration par contact avec du charbon activé pendant une heure afin d'éliminer les pigments tels que les chlorophylles et xanthophylles ;
4) Le mélange décoloré est séparé du résidu de charbon à l'aide d'une microfiltration (jusqu'à 1µm) ;
5) L'éthanol d'extraction est éliminé par évaporation. L'extrait est concentré jusqu'à atteindre un taux de matière sèche dans l'eau de l'ordre de 5% (poids/poids) ;
6) Le mélange est filtré à 0.2pm pour éliminer les particules fines et les bactéries résiduelles ;
7) Afin de préparer le levain servant à la fermentation de l'extrait, une partie de l'extrait obtenu avant l'étape 6) est concentrée à 30% de matière sèche dans l'eau puis 10% en poids de levures sèches Saccharomyces cerevisiae var. bayanus y sont ajoutés. Le mélange est agité durant 2 heures à 37°C. Ce mélange constitue le levain.
8) 2% de levain en poids sont ajoutés au mélange issu de l'étape 6)
9) Après 24 à 48 heures de fermentation à 37°C sous agitation et dans l'obscurité, l'extrait fermenté est laissé décanter une nuit pour éliminer les bulles de gaz formées.
10) L'extrait fermenté est ensuite filtré à 1µm, puis dilué pour obtenir un extrait contenant 1% de matière sèche. 0.7% de phénoxyéthanol ou 20% de 1,3-propanediol sont ajoutés pour une meilleure conservation et le tout est filtré à 0.2pm.

Les teneurs de l'extrait des fleurs de lys sont données dans le tableau 1 ci-dessous.

**Tableau 1 : Composition d'un extrait de Lys selon l'invention**

| **Fleurs de lys** *(Lilium candidum)* | | | | | |
|---|---|---|---|---|---|
| **Famille moléculaire** | **Molécules** | **Extrait conventionnel sec** | **Extrait conventionnel dilué à 1%** | **Extrait fermenté sec** | **Extrait fermenté dilué à 1%** |
| Saccharides | Fructose, Glucose, Sucrose | 58,00% | 0,58% | 0,00% | 0,00% |
| Azotés et autres | Lilidine glucoside, Glucopyranosyl glycerol, Acide hopanténique | 15,30% | 0,15% | 48,60% | 0,48% |
| Flavonoïdes et autres polyphénols | Kaempferol di et triglucosides, Quercetine diglucosides, Helionosides A, Regaloside A | 3,50% | 0,03% | 9,00% | 0,09% |
| Lipides | Acides gras C16:0, C18:0, C18:1, C18:2, C18:3 | 3,00% | 0,03% | 0,00% | 0,00% |
| Autres | Autres | 19,70% | 0,20% | 42,40% | 0,42% |

Comme démontré ci-dessus, le procédé selon la présente invention permet d'éliminer complètement les sucres simples d'un extrait de fleurs de lys. Le procédé selon l'invention permet également d'augmenter fortement la teneur en polyphénols de l'extrait.

### EXEMPLE 2 - La cinétique de fermentation des levures sèches de Saccharomyces cerevisiae var. bayanus Fizz+, Divine et 18-2007 permettent une fermentation optimale des extraits de fleurs.

Une étude cinétique de fermentation a permis de déterminer les levures qui sont plus efficaces.

De manière générale, la fermentation des extraits de fleurs peut être subdivisée en trois étapes successives (M.H. AKIN, « Evolution du pH pendant la fermentation alcoolique de moûts de raisins : modélisation et interprétation métabolique », (2008) 136) :
- étape 1 : hydrolyse du sucrose en Fructose et Glucose (dure entre 15 et 30 minutes)
- étape 2 : début de consommation du Glucose (dure jusqu'à 5 heures)
- étape 3 : début de consommation du Fructose et consommation des autres monosaccharides (varie en fonction de la souche)

Afin de sélectionner la souche de levure permettant une fermentation optimale, nous avons évalué la vitesse de consommation des sucres totaux de l'étape 3 à partir de l'extrait de Lys avec diverses souches de levures commerciales.

Les pentes ont été obtenues par régression linéaire des teneurs relatives en sucres totaux. Plus la pente est faible plus la cinétique est importante (Figure 1).

**Tableau 2 : Comparaison des vitesses de consommation des sucres totaux pour différentes souches de levures.**

| | **Vitesse de consommation des sucres totaux** | **Pente** | **R²** |
|---|---|---|---|
| ***Souche référence Saccharomyces cerevisiae* non *bayanus (ref.)*** | 2.1%/h | -0.5 | 0.94 |
| **18-2007** | 4.7%/h | -1.13 | 0.98 |
| ***Fizz+*** | 6.8%/h | -1.64 | 0.79 |
| **Divine** | 5.3%/h | -1.28 | 0.73 |

Ainsi, les résultats de la Figure 1 et du Tableau 2 montrent que les souches Fizz+, Divine, 18-2007 et référence (ref.) permettent la consommation respectivement de 6.8%, 5.3%, 4.7% et 2.1% de l'ensemble des sucres de l'extrait de fleurs de Lys par heure.

Cette comparaison permet alors de classer les 4 souches selon leur vitesse de consommation des sucres de la façon suivante : Fizz+ > Divine > 18-2007 > référence non*-Bayanus*

La souche référence, qui n'appartient pas à la variété *Saccharomyces cerevisiae var. bayanus,* consomme les sucres totaux deux à trois moins vite que les souches Fizz+, Divine et 18-2007 qui elles appartiennent à la variété *Saccharomyces cerevisiae var. bayanus.*

Les levures *Saccharomyces cerevisiae var. bayanus* permettent d'obtenir une cinétique optimale de fermentation des extraits végétaux.

### Exemple 3 : Composition cosmétique

La composition suivante peut être préparée de façon classique pour l'homme du métier. Les quantités indiquées ci-dessous sont exprimées en pourcentages pondéraux. Les ingrédients en majuscules sont identifiés conformément à la dénomination INCI.

### Emulsion huile dans eau

| **INCI /TRADE NAME SUPPLIER** | **(% W/W)** |
|---|---|
| Jojoba esters | 1-10 |
| Camellia seed oil | 1-10 |
| Butyrospermum Parkii Butter (LIPEX SHEA) | 1-10 |
| Butyrospermum parkii butter (LIPEX SHEASOFT) | 1-10 |
| Shea Butter Ethyl Esters (LIPEX SHEALIGHT) | 1-10 |
| Butyrospernum parkii butter extract (LIPEX SHEA TRIS) | 1-10 |
| PHYTOSQUALAN | 0.5-7 |
| cetyl dimethicone (ABIL WAX 9801) | 0.1-7 |
| isostearyl isostearate (CRODAMOL ISIS-LQ) | 1-5 |
| cetyl alcohol & glyceryl stéarate & peg-75 stéarate & ceteth-20 & steareth-20 (EMULIUM DELTA) | 1-5 |
| sodium polyacrylate (COVACRYL MV 60) | 1-5 |
| silica & lauroyl lysine (AMILON) | 0.1-10 |
| Sodium hyaluronate | 0.01-3 |
| Glycerin | 1-30 |
| Polyquaternium-51 | 1-10 |
| Extrait obtenu selon l'invention | 0.001-10 |
| Adenosine | 0.1-0.5 |
| Niacinamide | 0.1-5 |
| Palmitoyl Tripeptide-1 & Palmitoyl Tetrapeptide-7 | 1-5 |
| Secale Cereale (Rye) Seed Extract | 1-5 |
| Ascorbyl glucoside | 0.001-5 |
| Glycols (Caprylyl Glycol and/or Pentylene Glycol and/or Butylène Glycol and/or propanediol) | 0,1-10 |
| Water | Qs 100 |

## Revendications

1. Procédé pour la préparation d'un extrait de fleur enrichi en polyphénols et/ou substantiellement dépourvu de sucres simples, comprenant une étape de fermentation d'un extrait de fleur avec une levure appartenant à la variété *Saccharomyces cerevisiae* var. *bayanus.*

2. Procédé selon la revendication 1 comprenant, avant l'étape de fermentation, la préparation d'un extrait de fleur au moyen des étapes suivantes :
a) l'extraction d'au moins une fleur avec au moins un solvant alcoolique et/ou de l'eau ;
b) la filtration du mélange obtenu en a) afin d'éliminer les résidus végétaux;
c) optionnellement, la décoloration du mélange obtenu à l'issue de l'étape b; et
d) l'élimination du solvant et la concentration de l'extrait obtenu.

3. Procédé selon l'une des revendications 1 ou 2, dans lequel ladite levure de variété *Saccharomyces cerevisiae* var. *bayanus* est choisie dans le groupe constitué des levures des souches IOC Fizz+, IOC Divine, IOC 18-2007 et des mélanges de celles-ci.

4. Procédé selon la revendication 3, dans lequel ladite levure de variété *Saccharomyces cerevisiae* var. *bayanus* est une levure de la souche IOC Fizz+.

5. Procédé selon la revendication 3, dans lequel ladite levure de variété *Saccharomyces cerevisiae* var. *bayanus* est une levure de la souche IOC Divine.

6. Procédé selon la revendication 3, dans lequel ladite levure de variété *Saccharomyces cerevisiae* var. *bayanus* est une levure de la souche IOC 18-2007.

7. Procédé selon l'une quelconque des revendications 1 à 6, dans lequel la fleur est une fleur de *Lilium candidum, Myosotis sylvatica, Sambucus sp., Camellia sp., Primula sp., Polianthes tuberosa, Jasminum grandiflorum, Plantago lanceolata, Cydonia oblonga, Prunus armeniaca* ou de *Plumeria sp..*

8. Procédé selon l'une quelconque des revendications 1 à 7, dans lequel l'étape de fermentation est effectuée au moyen d'un levain préparé à partir de la fermentation d'un extrait végétal concentré avec la souche de *Saccharomyces cerevisiae* var. *bayanus.*

## Patentansprüche

1. Verfahren zur Herstellung eines Blütenextrakts, der mit Polyphenolen angereichert und/oder im Wesentlichen frei von einfachen Zuckern ist, umfassend einen Schritt der Fermentation eines Blütenextrakts mit einer Hefe, die zur Varietät *Saccharomyces cerevisiae* var. *bayanus* gehört.

2. Verfahren nach Anspruch 1, das vor dem Fermentationsschritt die Herstellung eines Blütenextrakts mittels der folgenden Schritte umfasst:
a) Extraktion von wenigstens einer Blüte mit wenigstens einem alkoholischen Lösungsmittel und/oder Wasser;
b) Filtrieren der in a) erhaltenen Mischung, um pflanzliche Rückstände zu entfernen;
c) gegebenenfalls Entfärben der in Schritt b) erhaltenen Mischung; und
d) Entfernen des Lösungsmittels und Konzentrieren des erhaltenen Extrakts.

3. Verfahren nach einem der Ansprüche 1 oder 2, wobei die Hefevarietät *Saccharomyces cerevisiae* var. *bayanus* ausgewählt ist aus der Gruppe, die aus Hefen der Stämme IOC Fizz+, IOC Divine, IOC 18-2007 und Mischungen davon gebildet ist.

4. Verfahren nach Anspruch 3, wobei die Hefevarietät *Saccharomyces cerevisiae* var. *bayanus* eine Hefe des Stamms IOC Fizz+ ist.

5. Verfahren nach Anspruch 3, wobei die Hefevarietät *Saccharomyces cerevisiae* var. *bayanus* eine Hefe des Stamms IOC Divine ist.

6. Verfahren nach Anspruch 3, wobei die Hefevarietät *Saccharomyces cerevisiae* var. *bayanus* eine Hefe des Stamms IOC 18-2007 ist.

7. Verfahren nach einem der Ansprüche 1 bis 6, wobei die Blüte eine Blüte aus *Lilium candidum, Myosotis sylvatica, Sambucus sp., Camellia sp., Primula sp., Polianthes tuberosa, Jasminum grandiflorum, Plantago lanceolata, Cydonia oblonga*, *Prunus armeniaca* oder *Plumeria sp.* ist.

8. Verfahren nach einem der Ansprüche 1 bis 7, wobei der Fermentationsschritt mit Hilfe eines Sauerteigs durchgeführt wird, der aus der Fermentation eines konzentrierten Pflanzenextrakts mit dem Stamm *Saccharomyces cerevisiae* var. *bayanus* hergestellt wurde.

## Claims

1. Process for the preparation of a flower extract enriched in polyphenols and/or substantially free of simple sugars, comprising a step of fermentation of a flower extract with a yeast belonging to the variety *Saccharomyces cerevisiae* var. *bayanus.*

2. Process according to claim 1 comprising, prior to the fermentation step, the preparation of a flower extract by means of the following steps:
a) extraction of at least one flower with at least one alcoholic solvent and/or water;
b) filtration of the mixture obtained in a) in order to remove plant residues;
c) optionally, decolorization of the mixture obtained in step b); and
d) removal of the solvent and concentration of the extract obtained.

3. Process according to claim 1 or 2, wherein said yeast of the variety *Saccharomyces cerevisiae* var. *bayanus* is selected from the group consisting of yeasts of the strains IOC Fizz+, IOC Divine, IOC 18-2007 and mixtures thereof.

4. Process according to claim 3, wherein said yeast of the variety *Saccharomyces cerevisiae* var. *bayanus* is a yeast of the IOC Fizz+ strain.

5. Process according to claim 3, wherein said yeast of the variety *Saccharomyces cerevisiae* var. *bayanus* is a yeast of the IOC Divine strain.

6. Process according to claim 3, wherein said yeast of the variety *Saccharomyces cerevisiae* var. *bayanus* is a yeast of the IOC 18-2007 strain.

7. Process according to any one of claims 1 to 6, wherein the flower is a flower of *Lilium candidum*, *Myosotis sylvatica*, *Sambucus* sp., *Camellia* sp., *Primula* sp., *Polianthes tuberosa, Jasminum grandiflorum*, *Plantago lanceolata, Cydonia oblonga, Prunus armeniaca* or *Plumeria* sp.

8. Process according to any one of claims 1 to 7, wherein the fermentation step is carried out by means of a leaven prepared from the fermentation of a concentrated plant extract with the strain of *Saccharomyces cerevisiae* var. *bayanus.*
